# FASCICULE DE BREVET EUROPEEN

(11) **EP 2 041 173 B1**
(45) Date de publication et mention de la délivrance du brevet: **12.05.2010**
(21) Numéro de dépôt: 07788860.0
(22) Date de dépôt: 11.06.2007
(51) Int. Cl.: C07K 14/705, A61K 38/17

(54) **PEPTIDES A ACTIVITE ANTI-PROLIFERATIVE**
PEPTIDE MIT PROLIFERATIONSHEMMENDER WIRKUNG
PEPTIDES WITH ANTI-PROLIFERATIVE ACTIVITY

(30) Priorité: 12.06.2006 FR 0605193
(43) Date de publication de la demande: 01.04.2009
(73) Titulaire: IPSEN PHARMA, 92100 Boulogne-Billancourt (FR)
(72) Inventeur: FERRANDIS, Eric, 78470 Saint Remy Les Chevreuse (FR); CAMARA Y FERRER, José-Antonio, 75016 Paris (FR); MARIN, Jean-Grégoire, 91120 Palaiseau (FR)
(74) Mandataire: Bourgouin, André
(86) Numéro de dépôt international: PCT/FR2007/000955
(87) Numéro de publication internationale: WO 2007/144492

(56) Documents cités:
- WO-A-98/23741
- WO-A-2005/103079
- IDO Y ET AL: "Prevention of vascular and neural dysfunction in diabetic rats by C-peptide" SCIENCE, AMERICAN ASSOCIATION FOR THE ADVANCEMENT OF SCIENCE,, US, vol. 277, 25 juillet 1997 (1997-07-25), pages 563-566, XP002298804 ISSN: 0036-8075
- SONGOK EM ET AL.: "Identification of env CRF-10 among HIV Variants Circulating in Rural Western Kenya" AIDS RESEARCH AND HUMAN RETROVIRUSES, vol. 19, no. 2, 2003, pages 161-165, XP002413032 ISSN: 0889-2229

### SEQUENCE LISTING

<110> S.C.R.A.S.
   <120> peptides à activité anti-proliférative
   <130> RS 372
   <160> 3
   <170> Patentin version 3.3
<210> SEQ ID n° 1
   <211> 27
   <212> PRT
   <213> human
<400> 1
   Tyr Thr Val Leu Ser Val Gly Pro Gly Gly Leu Arg Ser Gly Arg Leu
   1 5 10 15
   Pro Leu Gln Pro Arg Val Gln Leu Asp Glu Arg
   20 25
<210> SEO ID n° 2
   <211> 15
   <212> PRT
   <213> human
<400> 2
   Ser Gly Arg Leu Pro Leu Gln Pro Arg Val Gln Leu Asp Glu Arg
   1 5 10 15
<210> SEQ ID n° 3
   <211> 6
   <212> PRT
   <213> human
<400> 3
   Val Gln Leu Asp Glu Arg
   1 5

## Description

La présente invention a pour objet des polypeptides et peptides à activité anti-proliférative ou un de leurs sels pharmaceutiquement acceptables, ainsi que leur utilisation pour la prévention ou le traitement du cancer.

Il existe de nombreux types de cancers. Parmi ceux-ci, on peut citer en particulier le neuroblastome.

Le neuroblastome est un cancer relativement rare du système nerveux sympathique qui affecte principalement les enfants. Le Neuroblastome est une tumeur maligne la plus commune chez les enfants, se développant à partir de cellules progénitrices de la crête neurale devant coloniser la médullo-surrénale et les ganglions sympathiques. Le neuroblastome est habituellement trouvé chez les bébés et les jeunes enfants. Environ 96% des cas de neuroblastomes apparaissent avant l'âge de 10 ans. La maladie apparaît généralement dans la glande médullaire adrénale ou dans d'autres sites du tissu nerveux sympathique, le site le plus commun étant dans l'abdomen aux environs de la glande adrénale. On peut aussi trouver des neuroblastomes dans la poitrine, le cou ou le pelvis. Les patients atteints d'un cancer du type neuroblastome ont une grande probabilité de se faire détecter. Les symptômes fréquents des neuroblastomes sont une pression provoquée par la tumeur ou une douleur osseuse. Les neuroblastomes peuvent aussi comprimer la moelle épinière provoquant une paralysie. On peut aussi constater des cas de fièvres, d'anémie, ou de pression sanguine élevée.

Le traitement du Neuroblastome est basé sur une approche multidisciplinaire associant essentiellement la chirurgie, la radiothérapie et la chimiothérapie. Parmi les traitements connus à ce jour, on peut citer les traitements utilisant le cyclophosphamide, la doxorubicine, l'étoposide, les sels de platine ou la vincristine.

Parmi les cancers, on peut également citer les leucémies.

La leucémie désigne un ensemble de cancers affectant le sang. L'adulte moyen possède environ cinq litres de sang, dont le rôle est de fournir les éléments nutritifs, l'oxygénation et l'évacuation des déchets. Le sang se compose de globules rouges, de globules blancs, de plaquettes et de plasma. Les globules blancs aident à combattre l'infection. Les plaquettes de sang forment des caillots empêchant la perte de sang au cours des blessures et hémorragies. Approximativement 55 % du sang est constitué de plasma, un liquide de couleur clair paille qui contient les cellules de sang et les plaquettes, et transporte l'alimentation provenant de la digestion et les hormones provenant des glandes dans l'organisme. Comme indiqué, les globules blancs sont responsables d'un mécanisme de défense. Il y a deux types principaux de globules blancs, les lymphocytes et les monocytes. Il y a deux types de lymphocytes, les lymphocytes B, impliqués dans la production des anticorps, et les lymphocytes T. Les lymphocytes T sont encore divisés en 3 groupes : les cellules T inflammatoires, qui recrutent des macrophages et des neutrophiles à l'emplacement de l'infection ou de tout autre tissu endommagé ; les lymphocytes cytotoxiques T, qui tuent les virus ayant infecté des cellules ; et cellules T-helper, qui augmentent la production des anticorps par les cellules B. La leucémie lymphoblastique aiguë est la leucémie la plus commune chez les enfants. C'est un cancer des globules blancs, spécifiquement des lymphocytes. Les cellules cancéreuses de leucémie sont des cellules de sang qui ne fonctionnent plus normalement. Par conséquent, les globules blancs ne peuvent pas aider le corps à combattre des infections. Pour cette raison, les enfants présentant une leucémie lymphoblastique aiguë ont fréquemment des infections et font des fièvres. Selon le nombre de cellules anormales et selon leur localisation, les patients présentant une leucémie peuvent avoir un certain nombre de symptômes. Les enfants présentant une leucémie lymphoblastique aiguë ont fréquemment de faibles quantités de globules rouges et de plaquettes saines. En conséquence, il n'y a pas assez de globules rouges pour porter l'oxygène aux organes, ce qui se traduit par une anémie, les patients pouvant sembler pâles avec une sensation de faiblesse et de fatigue. Quand il n'y a pas assez de plaquettes, les patients saignent et se blessent facilement. Certains des symptômes communs de la leucémie lymphoblastique aiguë incluent : fièvre ; fatigue ; infections fréquentes ; pâleur ; saignement facile ou meurtrissure ; taches rouges minuscules (appelées les petechiae) sous la peau ; et/ou douleur des os ou des articulations.

Les traitements connus à ce jour des leucémies sont entre autre les traitements utilisant l'Ara-C, l'Idarubicine, la Daunorubicine, le Melphalan ou le Busulphan.

Parmi les cancers, on peut également citer les cancers de la prostate.

Le cancer de la prostate est un cancer fréquent touchant exclusivement l'homme. La prostate fait partie du système reproducteur masculin. Une prostate saine est de la taille d'une châtaigne. La prostate est un organe situé immédiatement sous la vessie, en arrière de la symphyse pubienne et en avant du rectum. Elle entoure sur 3 à 4 cm l'urètre, canal évacuant les urines. La prostate est une glande qui produit une partie du liquide séminal. Une prostate élargie serrera l'urètre posant des problèmes urinaires en ralentissant ou en arrêtant l'écoulement de l'urine du réservoir souple au pénis. Plus de 70% de tous les cancers de la prostate sont diagnostiqués chez les hommes généralement au-delà de l'âge 65 ans. Bien que l'étiologie du cancer de la prostate soit inconnue, les facteurs de risque incluent l'environnement, la génétique et les antécédents familiaux. Le taux de mortalité pour le cancer de la prostate est au moins 2 fois plus élevé chez les hommes afro-américains que chez les hommes caucasiens. En raison du risque additionnel, un dépistage plus précoce pour le cancer de la prostate est recommandé pour les hommes afro-américains. Selon la société américaine du Cancer, les hommes agés de 50 ans et plus, et ceux au-delà de l'âge de 45 ans qui sont dans les groupes à haut risque, tels que les hommes afro-américains et les hommes avec des antécédents familiaux de cancer de la prostate, devraient avoir une analyse de sang avec dépistage de l'antigène spécifique de la prostate (PSA) et un examen rectal chaque année. Les symptômes du cancer de la prostate incluent généralement : des problèmes urinaires ; une incapacité d'uriner, ou une difficulté pour commencer ou stopper le flux d'urine ; la nécessité d'uriner fréquemment, particulièrement la nuit ; l'écoulement faible ou interrompu d'urine ; une douleur ou brûlure pendant la miction ; une difficulté pour obtenir une érection ; la présence de sang dans l'urine ou le sperme ; et des douleurs fréquentes dans le bas du dos, les hanches ou les cuisses.

Les traitements connus à ce jour pour traiter le cancer de la prostate sont entre autre l'utilisation d'un peptide, la triptoreline, qui est un analogue de la LHRH (Luteinizing Hormone Releasing Hormone), utilisé tant que la croissance des cellules tumorales est sous contrôle hormonal androgénique. Très fréquemment, après une longue période de traitement avec les analogues de la LHRH, les cellules tumorales perdent leur sensibilité au contrôle hormonal et deviennent alors hormono-résistantes.

La chimiothérapie est de préférence utilisée dans le cancer de la prostate quand celui-ci a évolué avec une extension extraprostatique et qu'il ne répond plus au traitement hormonal. Elle comprend l'utilisation de produits ou de mélanges de produits comme le docetaxel, le paclitaxel, l'estramustine/docetaxel, l'estramustine/etoposide, estramustine/vinblastine, et l'estramustine/paclitaxel.

Le traitement des cancers, tels que par exemple le neuroblastome, la leucémie, le cancer de la prostate, le cancer du sein, le mélanome ou le cancer colorectal, peut être local ou systémique. Les traitements locaux, tels que la chirurgie et le rayonnement, affectent les cellules cancéreuses de la tumeur et le secteur près de celle-ci. Les traitements systémiques, tels que la chimiothérapie, la thérapie hormonale, et la thérapie biologique, sont véhiculés par la circulation sanguine, atteignant les cellules cancéreuses partout dans le corps, mais également des cellules saines provoquant beaucoup d'effets indésirables.

WO 98/23741 divulgue des polypeptides correspondant à une forme mutée de la protéine LAG-3 pour le traitement des cancers.

Afin de répondre aux exigences des industriels, il est devenu nécessaire de trouver de nouveaux traitements du cancer et en particulier d'autres composés ayant une activité anti-cancéreuse.

Aussi le problème que se propose de résoudre l'invention est de fournir de nouveaux composés ayant une activité anti-cancéreuse.

De manière inattendue, les inventeurs ont mis en évidence que des peptides de séquence SEQ ID n° 1 ou SEQ ID n° 2 ou SEQ ID n° 3 possèdent une activité anti-proliférative.

Dans ce but la présente invention propose un polypeptide d'au plus 30 acides aminés comprenant au moins une séquence peptidique choisie parmi la séquence SEQ ID n° 1 ou SEQ ID n° 2 ou SEQ ID n° 3.

L'invention propose aussi en tant que médicament, le peptide de séquence SEQ ID n° 1 ou SEQ ID n° 2 ou SEQ ID n° 3, ainsi que le polypeptide décrit ci-dessus.

L'invention concerne également des compositions pharmaceutiques comprenant le polypeptide ou peptide selon l'invention.

Enfin, l'invention a également pour objet l'utilisation des polypeptides, peptides et composés selon l'invention pour traiter ou prévenir les cancers du colon, du rectum, du sein, des poumons, du pancréas, des testicules, du rein, de l'utérus, de l'ovaire, de la prostate, de la peau, des os, de la moelle épinière ainsi que les sarcomes, les carcinomes, les fibroadénomes, les neuroblastomes, les leucémies, les lymphomes, les mélanomes.

L'invention concerne également des anticorps dirigés contre les peptides ou les composés tels que définis ci-dessus, ou contre un peptide **caractérisé en ce que** les résidus arginine des séquences SEQ ID n°1 ou SEQ ID n° 2 ou SEQ ID n° 3 sont, en totalité ou en partie, supprimés, modifiés ou remplacés par d'autres acides aminés.

L'invention offre des avantages déterminants, en particulier les polypeptides, peptides ou les composés selon l'invention étant très spécifiques des cellules ciblées, ce qui diminue les effets secondaires.

Avantageusement les polypeptides, les peptides ou les composés selon l'invention ou leurs sels présentent une solubilité accrue dans les milieux biologiques, en particulier dans les milieux aqueux.

L'invention a pour avantage de pouvoir être mise en oeuvre dans toutes industries, notamment l'industrie pharmaceutique, vétérinaire ou cosmétique.

D'autres avantages et caractéristiques de l'invention apparaîtront clairement à la lecture de la description et des exemples donnés à titre purement illustratifs et non limitatifs qui vont suivre.

Par l'expression cancer, on entend au sens de l'invention tout type de cancer, c'est à dire invasif, non invasif, infiltrant, hormonal, non hormonal, localisé ou métastatique.

Par l'expression peptide, on entend au sens de l'invention une séquence en acides aminés consistant en au plus 30 résidus acides aminés. Ces acides aminés forment ou non une séquence continue, linéaire ou non, branchée ou non. Un peptide selon l'invention peut également comprendre des peptides variants tels que définis ci-après.

L'invention a pour objet un peptide d'au plus 30 acides aminés comprenant au moins une séquence peptidique choisie parmi la séquence SEQ ID n° 1, SEQ ID n° 2 ou SEQ ID n° 3.

L'invention a également pour objet un peptide d'au plus 25 acides aminés comprenant au moins une séquence peptidique choisie parmi la séquence SEQ ID n° 2 ou SEQ ID n° 3.

Plus particulièrement, le peptide selon l'invention est un peptide d'au plus 18 acides aminés comprenant au moins la séquence peptidique SEQ ID n° 2 ou SEQ ID n° 3.

Encore plus particulièrement, le peptide selon l'invention est un peptide d'au plus 10 acides aminés comprenant au moins la séquence peptidique SEQ ID n° 3.

L'invention a également pour objet un peptide de séquence SEQ ID n° 1 reproduite ci-après :
Tyr Thr Val Leu Ser Val Gly Pro Gly Gly Leu Arg Ser Gly Arg Leu Pro Leu Gln Pro Arg Val Gln Leu Asp Glu Arg.

L'invention a également pour objet un peptide de séquence SEQ ID n° 2 reproduite ci-après :
Ser Gly Arg Leu Pro Leu Gln Pro Arg Val Gln Leu Asp Glu Arg.

L'invention a également pour objet un peptide de séquence SEQ ID n° 3 reproduite ci-après :
Val Gln Leu Asp Glu Arg .

Selon une variante de l'invention, le polypeptide, le peptide ou le composé selon l'invention peut présenter des modifications.

Selon un mode de réalisation particulier de l'invention, le polypeptide ou le peptide selon l'invention présente des résidus arginine des séquences SEQ ID n° 1 ou SEQ ID n° 2 ou SEQ ID n° 3 qui sont, en totalité ou en partie, supprimés, modifiés ou remplacés par d'autres acides aminés. Certains peptides selon l'invention contiennent des substitutions conservatrices d'arginine. Une substitution conservatrice est une substitution dans laquelle un acide aminé est substitué par un autre acide aminé ayant les mêmes propriétés, comme celles déterminées par l'homme du métier qui n'attend aucun changement dans la structure secondaire, ainsi que dans la nature hydropathique du polypeptide ou peptide. Les substitutions d'acide aminé peuvent généralement être réalisées sur la base de similarité de polarité, de charge, de solubilité, d'hydrophobicité, d'hydrophylicité, et/ou de la nature amphipathique des résidus. Des acides aminés chargés positivement incluent la lysine et l'arginine. D'autres groupes d'acides aminés qui peuvent représenter des changements conservateurs sont notamment les suivants :

Un peptide selon l'invention peut également, ou alternativement, contenir des changements non conservateurs.

La présente invention inclut également des polypeptides ou peptides avec ou sans motifs de glycosylation. Par exemple, le polypeptide ou le peptide selon l'invention peut être glycosylé ou comprendre des résidus de glycosylation comme des acétyls. Dans ce cas des groupes acétyls sont greffés sur la chaîne peptidique.

La présente invention inclut également des polypeptides ou peptides portant une ou plusieurs mutations de site de clivage par les protéases permettant une résistance aux protéases et donc une stabilité accrue dans la circulation.

Le polypeptide ou le peptide selon l'invention peut également comprendre une fonction amide à l'une des ses extrémités, ou à ses deux extrémités.

De préférence, l'invention a pour objet un composé de formule ou peptide amidé de séquence SEQ ID n° 1 reproduite ci-après :
NH₂ -Tyr Thr Val Leu Ser Val Gly Pro Gly Gly Leu Arg Ser Gly Arg Leu Pro Leu Gin Pro Arg Val Gln Leu Asp Glu Arg- CO -NH₂

De préférence, l'invention a également pour objet un composé de formule ou un peptide amidé de séquence SEQ ID n° 2 reproduite ci-après :
NH₂ -Ser Gly Arg Leu Pro Leu Gln Pro Arg Val Gln Leu Asp Glu Arg - CO - NH₂

De préférence, l'invention a également pour objet un composé de formule ou un peptide amidé de séquence SEQ ID n° 3 reproduite ci-après :
NH₂ -Val Gln Leu Asp Glu Arg- CO -NH₂.

Les modifications et variants décrits ci-dessus pour le polypeptide ou le peptide selon l'invention sont également valables pour le composé selon l'invention.

De préférence le polypeptide, le peptide ou le composé selon l'invention est linéaire, mais il est possible qu'il se présente sous forme circulaire ou hélicoïdale.

Le polypeptide, le peptide ou le composé selon l'invention peut comprendre des acides aminés lévogyrs (L) ou dextrogyrs (D) ou les deux.

De préférence, le polypeptide, le peptide ou le composé selon l'invention comprend des acides aminés lévogyrs.

L'invention a également pour objet le peptide de séquence SEQ ID n° 1 ou SEQ ID n° 2 ou SEQ ID n° 3 pour utilisation comme médicament.

L'invention a également pour objet le polypeptide selon l'invention décrite ci-dessus pour utilisation comme médicament.

L'invention a également pour objet le composé selon l'invention décrite ci-dessus pour utilisation comme médicament.

Selon certains aspects de l'invention, les polypeptides, peptides, composés, anticorps selon l'invention peuvent être incorporés dans des compositions pharmaceutiques. Les compositions pharmaceutiques comprennent un ou plusieurs de ces produits et un ou des excipients (transporteurs) pharmaceutiquement acceptables.

Aussi l'invention concerne des compositions pharmaceutiques comprenant au moins un polypeptide, un peptide ou un composé selon l'invention.

L'invention concerne aussi des anticorps dirigés contre les polypeptides, les peptides ou les composés selon l'invention. Il peut s'agir de sérums contenant des anticorps, ces sérums étant soit polyclonaux ou monoclonaux. Selon une variante de l'invention il peut s'agir d'un fragment d'un anticorps, ce fragment comprenant le site de liaison à l'antigène qui fixe spécifiquement le polypeptide, le peptide ou le composé tel que décrit précédemment, et en particulier un anticorps monoclonal, ou un fragment de celui-ci, qui fixe spécifiquement le peptide de séquence SEQ. ID. n° 1 , n° 2 ou n° 3.

Les anticorps peuvent être préparés par n'importe quelle technique disponible à l'homme du métier (cf. Harlow et Lane, Antibodies. A laboratory Manual, Cold Spring Harbor Laboratory, 1988). En général, les anticorps peuvent être produits par des techniques de culture cellulaire incluant la génération d'anticorps monoclonaux ou via des transfections de gènes d'anticorps dans des cellules hôtes de bactéries ou de mammifères afin de produire des anticorps recombinants.

Parmi d'autres techniques, on préférera employer celles décrites ci-après. Un immunogène contenant le polypeptide, le peptide ou le composé selon l'invention est injecté chez un groupe de mammifères (par exemple souris, rats, lapins, moutons ou chèvres). Dans cette étape, les polypeptides, les peptides ou les composés de la présente invention peuvent servir d'immunogènes sans modification.

Alternativement, une réponse immunitaire supérieure peut être induite si le polypeptide, le peptide ou le composé est joint à une protéine de transport comme l'albumine de sérum bovin ou l'hémocyanine de patelle.

L'immunogène est injecté chez l'animal hôte, de préférence selon un schéma prédéterminé, et les animaux sont saignés périodiquement. Des anticorps polyclonaux spécifiques du polypeptide, du peptide ou du composé selon l'invention peuvent ainsi être purifiés à partir de tels antisérums, par exemple par chromatographie d'affinité en utilisant le polypeptide ou peptide couplé à un support solide adéquat.

Selon un autre aspect de la présente invention, les polypeptides, les peptides ou les composés ainsi que leurs variants selon l'invention, peuvent être utilisés dans le traitement des maladies prolifératives.

Ainsi les polypeptides, les peptides ou les composés, ainsi que leurs variants selon l'invention, peuvent être utilisés dans le traitement des cellules cancéreuses, pré-cancéreuses ainsi que les cancers. En particulier, les polypeptides, les peptides ou les composés peuvent être utilisés pour inhiber la croissance et induire une modulation de la prolifération cellulaire en thérapie anticancéreuse, comme par exemple dans des tumeurs spécifiques du sein ou de la prostate.

De tels polypeptides, peptides ou composés peuvent également être utilisés pour la thérapie de nombreux carcinomes incluant les mélanomes, les formes multiples de glioblastomes, les carcinomes du poumon ainsi que les cancers colorectaux. Des agents qui activent l'expression de tels polypeptides, peptides ou composés peuvent également être employés dans le cadre de ces thérapies.

Selon ces aspects de l'invention, les polypeptides, les peptides ou les composés, ainsi que leurs variants selon l'invention, sont de préférence incorporés dans des compositions pharmaceutiques comme décrites ci-dessus.

Les patients adéquats pour la thérapie sont tous les animaux à sang chaud, et de préférence l'être humain. Un patient éligible pour une thérapie selon l'invention peut ou non être diagnostiqué comme étant affecté par un cancer. Autrement dit, les compositions pharmaceutiques décrites ci-dessus peuvent ainsi être utilisées pour inhiber le développement d'un cancer à différents stades de la maladie (pour prévenir l'apparition d'un cancer ou pour traiter un patient affecté par un cancer).

De manière particulière, l'invention a pour objet l'utilisation du polypeptide, du peptide ou du composé décrit ci-dessus, ainsi que leurs variants, pour traiter ou prévenir les cancers du colon, du rectum, du sein, des poumons, du pancréas, des testicules, du rein, de l'utérus, de l'ovaire, de la prostate, de la peau, des os, de la moelle épinière ainsi que les sarcomes, les carcinomes, les fibroadénomes, les neuroblastomes, les leucémies, les lymphomes, les mélanomes.

Les compositions pharmaceutiques de la présente invention seront administrées de manière appropriée pour chaque cancer spécifique à traiter.

Le polypeptide, le peptide ou le composé selon l'invention ou son sel peut se présenter sous forme solide, par exemple des poudres, des granules, des comprimés, des gélules, des liposomes ou des suppositoires. Les supports solides appropriés peuvent être, par exemple, le phosphate de calcium, le stéarate de magnésium, les sucres, le lactose, la dextrine, l'amidon, la gélatine, la cellulose, la cellulose de méthyle, la cellulose carboxyméthyle de sodium et la polyvinylpyrrolidine.

Le polypeptide, le peptide ou le composé selon l'invention ou son sel utilisé selon l'invention peut aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Le polypeptide, le peptide ou le composé selon l'invention ou son sel utilisé selon l'invention peut aussi se présenter sous forme semi-liquide, par exemple des gels.

L'administration du polypeptide, du peptide ou du composé selon l'invention ou son sel utilisé selon l'invention pourra se faire par voie topique, systémique, orale, parentérale, par injection intramusculaire, intraveineuse, sous-cutanée, intra-pérotonéale etc. On peut aussi envisager une administration locale, au niveau d'une tumeur cancéreuse ou suspectée cancéreuse.

La dose du produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et les propriétés pharmacologiques et pharmacodynamiques des polypeptides, des peptides ou des composés selon l'invention.

A titre indicatif, la dose d'administration envisagée pour un médicament selon l'invention est comprise entre 0,1 µg et 1 g / kg suivant le type de composé actif utilisé.

La figure 1 présente l'effet des composés 1, 2 et 3 sur la prolifération des cellules tumorales de neuroblastomes humains SHSY-5Y.

La figure 2 présente l'effet du composé 1 sur la prolifération des cellules tumorales de neuroblastomes humains SHSY-5Y xénogreffées chez la souris athymique.

La figure 3 présente l'absence de toxicité du composé 1 chez la souris athymique xénogreffée.

Les exemples suivants illustrent l'invention sans en limiter la portée.

### Etude pharmacologique des produits de l'invention

Dans les exemples ci-après, on désigne par :
- « Composé 1 » le composé de formule ou peptide amidé de séquence SEQ ID n° 1 reproduite ci-après :
   NH₂ -Tyr Thr Val Leu Ser Val Gly Pro Gly Gly Leu Arg Ser Gly Arg Leu Pro Leu Gln Pro Arg Val Gln Leu Asp Glu Arg-CO-NH₂ ;
- « Composé 2 » le composé de formule ou un peptide amidé de séquence SEQ ID n° 2 reproduite ci-après :
   NH₂ -Ser Gly Arg Leu Pro Leu Gln Pro Arg Val Gln Leu Asp Glu Arg -CO- NH₂ ;

- « Composé 3 » le composé de formule ou un peptide amidé de séquence SEQ ID n° 3 reproduite ci-après :
   NH₂ -Val Gln Leu Asp Glu Arg-CO-NH₂.

### 1/ Synthèse des composés 1, 2 et 3 :

La synthèse des composés 1, 2 et 3 a été réalisée en utilisant les techniques connues de synthèse des peptides.

La synthèse des composés 1, 2 et 3 a été réalisée en utilisant la chimie dite F-moc. La résine utilisée est la résine rinc-amide, fournie par Applied Biosystems. Les acides aminés proviennent également du même fournisseur. La méthode d'activation utilisée est la méthode dite HAUT/DIPEA. Le clivage de la résine et des protections latérales se fait grâce à la méthode suivante : 92% de TFA, 4% H₂O, 2,5% EDT et 1,5% TIS. La purification est réalisée par chromatographie liquide haute pression (ou HPLC) phase inverse, en utilisant des colonnes aquapores C8, 250 X 10 mn, 20 µm.

### 2/ test in vitro de prolifération cellulaire sur cellules du type SHSY-5Y avec les composés 1, 2 et 3 :

Des cellules cancéreuses de neuroblastome humain SHSY-5Y (Code ATCC :CRL-2266 sont cultivées dans du milieu DMEM (milieu de Eagle modifié par Dulbeco) contenant 100 U/ml de pénicilline et 100 µg/ml de sulfate de streptomycine, complémentée avec du sérum de veau foetal à 10 %. Les cellules sont sous-cultivées 24 h avant l'addition de concentrations croissantes des composés 1, 2 et 3 (0,001/0,01/0,1/1/10/100/1000 nM).

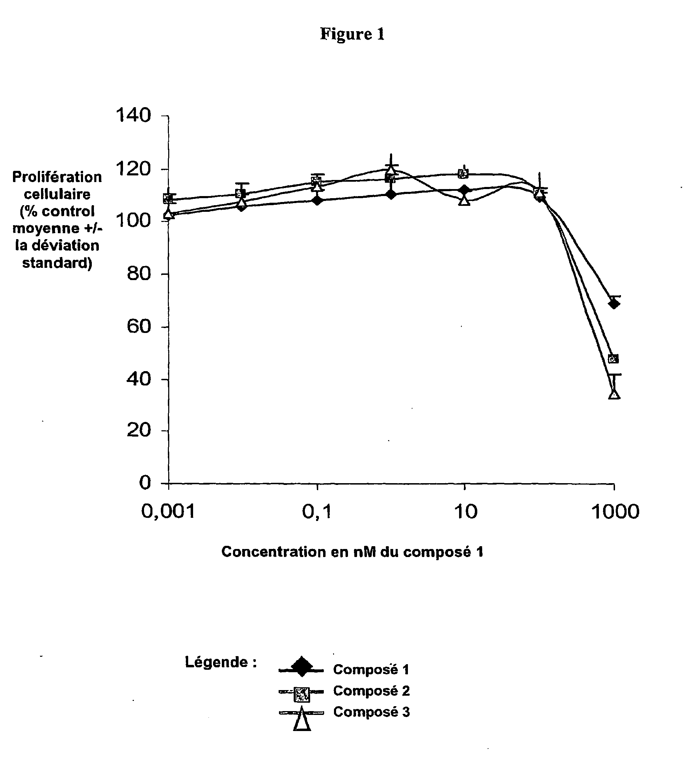

Les résultats sont reproduits en figure 1, décrivant l'effet des composés 1, 2 et 3 sur le pourcentage du nombre de cellules SHSY-5Y.

Les 3 composés montrent une forte activité anti-proliférative à la dose de 1000 nM sur les cellules SHSY-5Y.

### 3/ test in vivo de prolifération cellulaire sur cellules du type SHSY-5Y avec le composé 1 :

Des cellules cancéreuses de neuroblastomes humains SHSY-5Y (Code ATCC : CRL-2266) sont cultivées dans du milieu DMEM (milieu de Eagle modifié par Dulbeco) contenant 100 U/ml de pénicilline et 100 µg/ml de sulfate de streptomycine, complémenté avec du sérum de veau foetal à 10 %. Les cellules ainsi cultivées sont ensuite xénogreffées par voie sous-cutanée dans le flanc de souris balb-c athymiques NCr-nu/nu, âgées de 4 à 6 semaines.

Après un temps de latence de plusieurs jours suivant la greffe, les cellules SHSY-5Y proliférent et forment une tumeur de petite taille (environ 70 mm3) palpable sous le flanc de l'animal.

Le composé 1 est, à ce stade de croissance de la tumeur, injecté par voie intra-péritonnéale aux doses de 5 et 10 mg/kg.

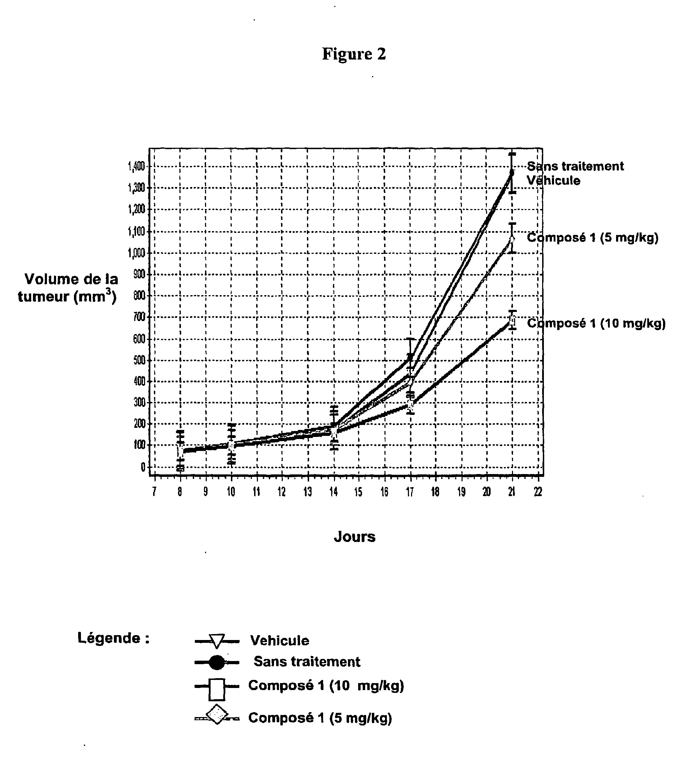

Les résultats sont reproduits en figure 2 décrivant l'effet du composé 1 sur la cinétique de croissance et le volume tumoral.

Le composé 1 présente une activité anti-tumorale dose-dépendante, liée à son activité anti-proliférative. En effet avec la dose de 5 mg/kg, on observe un ralentissement de la cinétique de croissance de la tumeur avec une diminution de la taille de la tumeur d'environ 20 % après 21 jours.

Cet effet est plus marqué lorsque le composé 1 est administré à la dose de 10 mg/kg pour laquelle on observe un très net ralentissement de la cinétique de croissance de la tumeur associé à une diminution de la taille de la tumeur d'environ 50 % après 21 jours.

### 4/ test de toxicité du composé 1 :

Des cellules cancéreuses de neuroblastome humain SHSY-5Y (Code ATCC : CRL-2266) sont cultivées dans du milieu DMEM (milieu de Eagle modifié par Dulbeco) contenant 100 U/ml de pénicilline et 100 µg/ml de sulfate de streptomycine, complémenté avec du sérum de veau foetal à 10 %. Les cellules ainsi cultivées sont ensuite xénogreffées en sous-cutanée dans le flanc de souris athymiques.

Après un temps de latence de plusieurs jours, les cellules SHSY-5Y proliférent et forment une tumeur palpable, de petite taille (environ 70 mm3).

Le composé 1 est, à ce stade de croissance de la tumeur, injecté par voie intra-péritonnéale aux doses de 5 et 10 mg/kg.

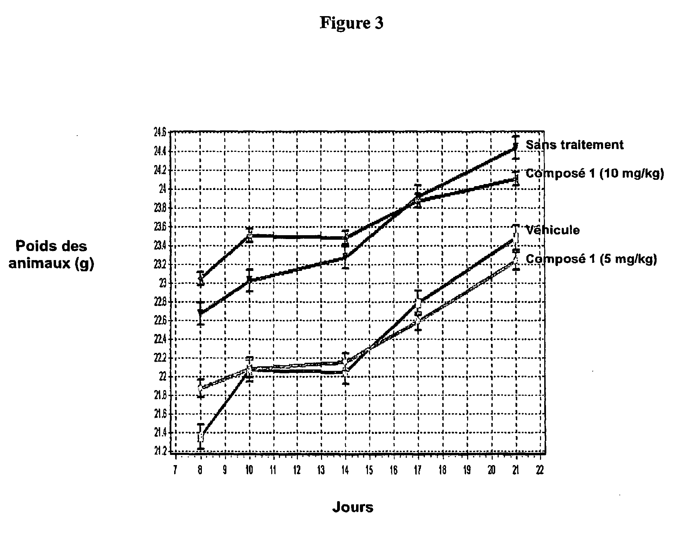

Les résultats sont reproduits en figure 3 décrivant l'évolution du poids des animaux en fonction du temps, qui est un indicateur de la toxicité éventuelle des produits testés dans ce modèle de souris athymique.

Le composé 1 ne présente aucun effet sur le poids des animaux quelle que soit la dose testée du composé 1 (5 et 10 mg/kg). Ce résultat indique l'absence de toxicité apparente de ce produit.

## Revendications

1. Peptide d'au plus 30 acides aminés comprenant au moins une séquence peptidique choisie parmi la séquence SEQ ID n° 1, SEQ ID n° 2 ou SEQ ID n° 3.

2. Peptide de séquence SEQ ID n° 1.

3. Peptide de séquence SEQ ID n°2.

4. Peptide de séquence SEQ ID n°3.

5. Peptide selon l'une des revendications précédentes **caractérisé en ce que** les résidus arginine des séquences SEQ ID n° 1 ou SEQ ID n° 2 ou SEQ ID n° 3 sont, en totalité ou en partie, supprimés, modifiés ou remplacés par d'autres acides aminés.

6. Peptide selon l'une des revendications précédentes **caractérisé en ce qu'**il comprend une fonction amide à l'une des ses extrémités.

7. Peptide selon l'une des revendications précédentes pour son utilisation comme médicament.

8. Peptide de séquence SEQ ID n° 1 ou SEQ ID n° 2 ou SEQ ID n° 3 pour utilisation comme médicament.

9. Composé de formule suivante :
NH₂ -Tyr Thr Val Leu Ser Val Gly Pro Gly Gly Leu Arg Ser Gly Arg Leu Pro Leu Gln Pro Arg Val Gln Leu Asp Glu Arg- CO -NH₂.

10. Composé de formule suivante :
NH₂ -Ser Gly Arg Leu Pro Leu Gln Pro Arg Val Gln Leu Asp Glu Arg - CO - NH₂

11. Composé de formule suivante :
NH₂ -Val Gln Leu Asp Glu Arg- CO -NH₂.

12. Composé selon l'une des revendications 9 à 11 pour utilisation comme médicament.

13. Composition pharmaceutique comprenant au moins un peptide ou un composé selon l'une des revendications 1 à 6 ou 9 à 11.

14. Anticorps dirigés contre les peptides ou les composés selon l'une des revendications 2 à 4 ou 9 à 11, ou contre un peptide **caractérisé en ce que** les résidus arginine des séquences SEQ ID n°1 ou SEQ ID n° 2 ou SEQ ID n° 3 sont, en totalité ou en partie, supprimés, modifiés ou remplacés par d'autres acides aminés.

15. Peptide ou composé selon l'une des revendications 1 à 6 ou 9 à 11 pour utilisation comme médicament dans le traitement des maladies prolifératives.

16. Peptide ou composé selon l'une des revendications 1 à 6 ou 9 à 11 pour utilisation comme médicament dans le traitement des cellules cancéreuses, pré-cancéreuses ainsi que les cancer.

17. Peptide ou composé selon l'une des revendications 1 à 6 ou 9 à 11 pour utilisation comme médicament pour traiter ou prévenir les cancers du colon, du rectum, du sein, des poumons, du pancréas, des testicules, du rein, de l'utérus, de l'ovaire, de la prostate, de la peau, des os, de la moelle épinière ainsi que les sarcomes, les carcinomes, les fibroadénomes, les neuroblastomes, les leucémies, les lymphomes, les mélanomes.

## Claims

1. Peptide with at most 30 amino acids comprising at least one peptide sequence chosen from the sequence SEQ ID No. 1, SEQ ID No. 2 or SEQ ID No. 3.

2. Peptide of sequence SEQ ID No. 1.

3. Peptide of sequence SEQ ID No.2.

4. Peptide of sequence SEQ ID No.3.

5. Peptide according to one of the preceding claims **characterized in that** the arginine residues of sequences SEQ ID No. 1 or SEQ ID No. 2 or SEQ ID No. 3 are, wholly or partially, deleted, modified or replaced by other amino acids.

6. Peptide according to one of the preceding claims **characterized in that** it comprises an amide function at one of its ends.

7. Peptide according to one of the preceding claims for its use as a medicament.

8. Peptide of sequence SEQ ID No. 1 or SEQ ID No. 2 or SEQ ID No. 3 for use as a medicament.

9. Compound of the following formula:
NH₂ -Tyr Thr Val Leu Ser Val Gly Pro Gly Gly Leu Arg Ser Gly Arg Leu Pro Leu Gln Pro Arg Val Gin Leu Asp Glu Arg- CO -NH₂.

10. Compound of the following formula:
NH₂ -Ser Gly Arg Leu Pro Leu Gln Pro Arg Val Gln Leu Asp Glu Arg - CO - NH₂

11. Compound of the following formula:
NH₂ -Val Gln Leu Asp Glu Arg- CO -NH₂.

12. Compound according to one of claims 9 to 11, for use as a medicament.

13. Pharmaceutical composition comprising at least one peptide or a compound according to one of claims 1 to 6 or 9 to 11.

14. Antibodies directed against the peptides or compounds according to one of claims 2 to 4 or 9 to 11, or against a peptide **characterized in that** the arginine residues of sequences SEQ ID No. 1 or SEQ ID No. 2 or SEQ ID No. 3 are, wholly or partially, deleted, modified or replaced by other amino acids.

15. Peptide or compound according to one of claims 1 to 6 or 9 to 11 for use as a medicament in the treatment of proliferative diseases.

16. Peptide or compound according to one of claims 1 to 6 or 9 to 11 for use as a medicament in the treatment of cancerous and pre-cancerous cells as well as cancers.

17. Peptide or compound according to one of claims 1 to 6 or 9 to 11 for use as a medicament for treating or preventing cancers of the colon, rectum, breast, lungs, pancreas, testicles, kidney, uterus, ovary, prostate, skin, bones, spinal cord as well as sarcomas, carcinomas, fibroadenomas, neuroblastomas, leukaemias, lymphomas and melanomas.

## Patentansprüche

1. Peptid mit höchstens 30 Aminosäuren, umfassend wenigstens eine Peptidsequenz, ausgewählt aus der Sequenz SEQ-ID Nr. 1, SEQ-ID Nr. 2 oder SEQ-ID Nr. 3.

2. Peptid mit der Sequenz SEQ-ID Nr. 1.

3. Peptid mit der Sequenz SEQ-ID Nr. 2.

4. Peptid mit der Sequenz SEQ-ID Nr. 3.

5. Peptid gemäß einem der vorhergehenden Ansprüche, **dadurch gekennzeichnet, dass** die Argininreste der Sequenzen SEQ-ID Nr. 1 oder SEQ-ID Nr. 2 oder SEQ-ID Nr. 3 vollständig oder teilweise entfernt, modifiziert oder durch andere Aminosäuren ersetzt sind.

6. Peptid gemäß einem der vorhergehenden Ansprüche, **dadurch gekennzeichnet, dass** es an einem seiner Enden eine Amidfunktion enthält.

7. Peptid gemäß einem der vorhergehenden Ansprüche zur Verwendung als Arzneimittel.

8. Peptid der Sequenz SEQ-ID Nr. 1 oder SEQ-ID Nr. 2 oder SEQ-ID Nr. 3 zur Verwendung als Arzneimittel.

9. Verbindung mit der folgenden Formel:
NH₂ -Tyr Thr Val Leu Ser Val Gly Pro Gly Gly Leu Arg Ser Gly Arg Leu Pro Leu Gln Pro Arg Val Gln Leu Asp Glu Arg- CO -NH₂.

10. Verbindung mit der folgenden Formel:
NH₂ -Ser Gly Arg Leu Pro Leu Gln Pro Arg Val Gln Leu Asp Glu Arg - CO - NH₂.

11. Verbindung mit der folgenden Formel:
NH₂ -Val Gln Leu Asp Glu Arg- CO -NH₂.

12. Verbindung gemäß einem der Ansprüche 9 bis 11 zur Verwendung als Arzneimittel.

13. Pharmazeutische Zusammensetzung, umfassend wenigstens ein Peptid oder eine Verbindung gemäß einem der Ansprüche 1 bis 6 oder 9 bis 11.

14. Antikörper, gerichtet gegen die Peptide oder die Verbindungen gemäß einem der Ansprüche 2 bis 4 oder 9 bis 11 oder gegen ein Peptid, das **dadurch gekennzeichnet ist, dass** die Argininreste der Sequenzen SEQ-ID Nr. 1 oder SEQ-ID Nr. 2 oder SEQ-ID Nr. 3 vollständig oder teilweise entfernt, modifiziert oder durch andere Aminosäuren ersetzt sind.

15. Peptid oder Verbindung gemäß einem der Ansprüche 1 bis 6 oder 9 bis 11 zur Verwendung als Arzneimittel zur Behandlung proliferativer Erkrankungen.

16. Peptid oder Verbindung gemäß einem der Ansprüche 1 bis 6 oder 9 bis 11 zur Verwendung als Arzneimittel zur Behandlung von Krebszellen, Vorläufern von Krebszellen sowie Karzinomen.

17. Peptid oder Verbindung gemäß einem der Ansprüche 1 bis 6 oder 9 bis 11 zur Verwendung als Arzneimittel zur Behandlung oder Prävention von Darmkrebs, Mastdarmkrebs, Brustkrebs, Lungenkrebs, Bauchspeicheldrüsenkrebs, Hodenkrebs, Nierenkrebs, Gebärmutterkrebs, Eierstockkrebs, Prostatakrebs, Hautkrebs, Knochenkrebs, Rückenmarkskrebs sowie Sarkomen, Karzinomen, Fibroadenomen, Neuroblastomen, Leukämien, Lymphomen, Melanomen.
